# EUROPÄISCHE PATENTANMELDUNG

(11) **EP 3 398 631 A1**
(43) Veröffentlichungstag der Anmeldung: **07.11.2018**
(21) Anmeldenummer: 18168988.6
(22) Anmeldetag: 24.04.2018
(51) Int. Cl.: A61M 5/178, A61M 5/28, A61M 5/31, A61J 1/06

(54) **SYSTEM UND VERFAHREN ZUM BEFÜLLEN VON BEHÄLTERN MIT NACHDRÜCKEN EINES VERSCHLUSSES**

(30) Priorität: 05.05.2017 DE 102017207573
(71) Anmelder: Bausch + Ströbel Maschinenfabrik Ilshofen GmbH + Co. KG, 74532 Ilshofen (DE)
(72) Erfinder: WALLISCH, Manuel, 74541 Vellberg (DE)
(74) Vertreter: Weickmann & Weickmann PartmbB

(57) **Zusammenfassung**

System zum Befüllen von Behältern (100) mit mindestens zwei Öffnungen (102, 104), wobei sich bei einer ersten (102) der mindestens zwei Öffnungen (102, 104) ein Kanal (110) mit einer Innenwand in der Form eines allgemeinen Zylinders anschließt, umfassend: eine Befüllstation (20) zum Befüllen des Behälters (100); eine Verschlussbetätigungsvorrichtung (30) zum Verschließen einer der ersten Öffnung (102) des Behälters (100) mit einem Verschlussmittel (106); und eine Steuervorrichtung, die ausgelegt ist, Ansteuersignale für Schritte eines Befüllvorgangs abzugeben, wobei das Verschlussmittel (106) beim Verschließen mit seinem Außenumfang dichtend an der Innenwand des Kanals (110) zur Anlage kommt und entlang des Kanals (110) bewegbar ist; wobei die Verschlussbetätigungsvorrichtung (30) Schiebemittel (32) umfasst, um das Verschlussmittel (106) entlang des Kanals (110) zu bewegen; und wobei die Steuervorrichtung ferner dazu ausgelegt ist, die Verschlussbetätigungsvorrichtung (30) dazu anzusteuern, das Verschlussmittel (106) nach einem Befüllen des Behälters (100) mittels der Befüllvorrichtung (22) entlang des Kanals (110) zu verschieben, so dass sich ein Volumen zwischen dem Verschlussmittel (106) und der zweiten Öffnung (104) des Behälters verkleinert. Die Erfindung betrifft ebenfalls ein entsprechendes Verfahren.

## Beschreibung

Die vorliegende Erfindung betrifft ein System zum Befüllen von Behältern mit mindestens zwei Öffnungen, wobei sich bei einer ersten der mindestens zwei Öffnungen ein Kanal mit einer inneren Wand in der Form eines allgemeinen Zylinders anschließt, insbesondere ein System gemäß dem Oberbegriff von Anspruch 1. Behälter können hierbei insbesondere Spritzen, Karpulen oder Ähnliches sein. Die vorliegende Erfindung betrifft ebenfalls ein entsprechendes Verfahren zum Befüllen eines Behälters.

Im medizinischen Bereich, insbesondere der Zahnmedizin, werden vielfach Einwegspritzen bzw. Karpulen verwendet, die zwei Öffnungen aufweisen. Vor dem Befüllvorgang wird in der einen ein Stopfen oder ein ähnlicher Verschluss platziert, der bei Anwendung bewegt werden kann, um durch die andere Öffnung die entsprechende medizinische Substanz herauszupressen.

Das Befüllen erfolgt dabei üblicherweise so, dass in der ersten Öffnung ein entsprechender Verschluss oder Stopfen eingesetzt wird, der einerseits in dem Kanal hinter der Öffnung verschiebbar ist und der andererseits dichtend an der Innenwand des Kanals anliegt. Im Folgenden wird dann durch die andere, zweite Öffnung die entsprechende Substanz eingeführt. Anschließend wird auch die zweite Öffnung verschlossen.

Da in dem Endprodukt möglichst keine Luftblasen enthalten sein sollten, wird üblicherweise der Stopfen genau so weit eingeführt, dass das verbleibende Volumen zur zweiten Öffnung hin exakt der gewünschten Flüssigkeitsmenge entspricht, so dass bei vollständiger Befüllung bis zur zweiten Öffnung hin die gewünschte Flüssigkeitsmenge enthalten ist.

Technisch ist eine entsprechend exakte Platzierung des Stopfens nicht stets möglich, so dass als Tolerenzausgleich der Stopfen im Mittel etwas tiefer platziert wird, als für die eigentliche Flüssigkeitsmenge bzw. Produktmenge erforderlich wäre. Dies erfolgt, damit eine bestimmte Mindestmenge garantiert ist.

Da darüber hinaus auch bei der Anwendung der Karpule nicht die gesamte Flüssigkeitsmenge herausgepresst werden kann, weil ein Teil im Schulter- und Halsbereich der Karpule bzw. Spritze nicht ausgedrückt werden kann, wird grundsätzlich eine größere Menge der Flüssigkeit eingefüllt als nominal erforderlich. Dies kann abhängig vom Produkt, der zulässigen Packmitteltoleranzen und der genauen Behälterform eine fünf bis zwanzig Prozent größere Menge als nominal erforderlich im Mittel bedeuten.

Alternativ ist es bekannt, sogar eine etwas größere Menge zu verwenden, aber anschließend nach Abschluss des Befüllvorgangs den Stopfen noch etwas zu verschieben, um aus dem vollständig befüllten Bereich zwischen Stopfen und zweiter Öffnung eine geeignete Menge überschüssiges Produkt abzusaugen. Dies verringert zwar die Menge, die über den nominal erforderlichen Wert hinaus abgefüllt wird, ist jedoch technisch aufwändig.

Vor diesem Hintergrund ist es Aufgabe der vorliegenden Erfindung, ein System und Verfahren zum Befüllen von entsprechenden Behältern anzugeben, das technisch leicht realisierbar ist und gleichzeitig den durch eine Überfüllung über den Nominalwert hinaus bedingten Produktverlust verringert.

Zur Lösung dieser Aufgabe wird gemäß der vorliegenden Erfindung ein System zum Befüllen von Behältern gemäß Anspruch 1 sowie ein Verfahren zum Befüllen von Behältern gemäß Anspruch 15 bereitgestellt. Vorteilhafte Ausgestaltungen sind Gegenstand der abhängigen Ansprüche.

Das erfindungsgemäße System zum Befüllen von Behältern mit mindestens zwei Öffnungen, wobei sich bei einer ersten der mindestens zwei Öffnungen ein Kanal mit einer Innenwand in der Form eines allgemeinen Zylinders anschließt, umfasst:
- eine Befüllstation mit einer Befüllvorrichtung und Mitteln zum Lagern eines Behälters in einer Befüllposition, in der der Behälter mittels der Befüllvorrichtung befüllbar ist;
- eine Verschlussbetätigungsvorrichtung, die ausgelegt ist, die erste Öffnung des Behälters mit einem Verschlussmittel zu verschließen, wobei die erste Öffnung in der Befüllposition tiefer positioniert ist als wenigstens eine zweite der mindestens zwei Öffnungen des Behälters; und
- eine Steuervorrichtung, die ausgelegt ist, Ansteuersignale für Schritte eines Befüllvorgangs abzugeben, wobei das Verschlussmittel wenigstens in einem Teilbereich einen Außenumfang aufweist, der so gestaltet ist, dass das Verschlussmittel beim Verschließen mit seinem Außenumfang dichtend an der Innenwand des Kanals zur Anlage kommt und so dass das Verschlussmittel entlang des Kanals bewegbar ist.

Das erfindungsgemäße System zeichnet sich gegenüber dem Bekannten dadurch aus, dass:
- die Verschlussbetätigungsvorrichtung Schiebemittel umfasst, mittels derer eine auf ein bereits in der ersten Öffnung angeordnetes Verschlussmittel wirkende Kraft ausübbar ist, um das Verschlussmittel entlang des Kanals weiter zu bewegen;
- die Steuervorrichtung ferner dazu ausgelegt ist, ein Befüllen des Behälters nach einem definierten Volumen zu beenden, das geringer ist als ein Volumen zwischen dem Verschlussmittel und der zweiten Öffnung des Behälters; und
- die Steuervorrichtung ferner dazu ausgelegt ist, die Verschlussbetätigungsvorrichtung dazu anzusteuern, nach einem Befüllen des Behälters mittels der Befüllvorrichtung das Verschlussmittel entlang des Kanals mittels des Schiebemittels zu verschieben, so dass sich ein Volumen zwischen dem Verschlussmittel und der zweiten Öffnung des Behälters verkleinert.

Durch das Verschieben bzw. Nachdrücken des Stopfens bzw. des Verschlussmittels kann überschüssiges Gas, im Allgemeinen Raumluft, aus dem Behälter hinausgeschoben werden. Dadurch verbleibt keine störende Luft im Behälter.

Gleichzeitig führt die Ungenauigkeit beim initialen Positionieren des Stopfens vor dem Befüllen nicht mehr dazu, dass eine größere Menge eingefüllt werden muss, um diese Ungenauigkeit auszugleichen. Stattdessen wird diese Ungenauigkeit durch das Verschieben des Stopfens oder Verschlussmittels nach dem Befüllen aber vor Verschluss der zweiten Öffnung ausgeglichen.

Das definierte Volumen ist hierbei im Allgemeinen die nominal erforderliche Füllmenge bzw. das Nennvolumen plus die Menge, die aufgrund der Form des Kopf- und Halsbereichs nicht bei Anwendung ausgedrückt werden kann. Hingegen kann die zusätzliche Menge aufgrund der Ungenauigkeit der initialen Positionierung des Verschlussmittels vermieden werden. Dadurch genügt im Allgemeinen eine Befüllung mit weniger als 110 % des erforderlichen Nennvolumens, bei bestimmten Behälterformen auch weniger als 105 %.

Damit kann gegenüber den bisherigen Systemen die erforderliche Befüllung pro Behälter reduziert werden, ohne dass ein komplexes Absaugsystem erforderlich ist.

Vorteilhafterweise ist die Steuervorrichtung ferner dazu ausgelegt, die Ansteuerung der Verschlussbetätigungsvorrichtung, das Verschlussmittel entlang des Kanals zu verschieben, einzustellen, bevor oder wenn die Differenz zwischen definiertem Volumen minus Volumen zwischen dem Verschlussmittel und der zweiten Öffnung des Behälters einen definierten absoluten oder relativen Wert erreicht oder unterschreitet.

Mit relativem Wert ist hier insbesondere gemeint, dass die Differenz im Vergleich zum Gesamtvolumen bzw. zum Füllmenge klein ist. Also beispielsweise, dass die Differenz nur 2 % des gesamten Volumens zwischen Verschlussmittel und zweiter Öffnung entspricht bzw. in anderen Worten, dass dieses Volumen zu 98 % mit der entsprechenden Flüssigkeit befüllt ist.

Ein solches Steuerverhalten ist insbesondere dadurch erreichbar, dass für bestimmte Behälterformen mithilfe von Messungs- und Erfahrungswerten ein bestimmter Verschiebeweg ermittelt wird, der zu dem entsprechenden Ergebnis führt. Denn ein längerer Verschiebeweg des Verschlussmittels entlang des Kanals entspricht einer größeren Volumenverringerung.

Insbesondere kann die Verschiebung aber auch andauern, bis die Differenz Null ist, also dass das gesamte Volumen zwischen Verschlussmittel und zweiter Öffnung lediglich die entsprechende Flüssigkeit oder Substanz enthält.

Bevorzugt ist das Verschlussmittel ein Stopfen in Form eines allgemeinen Zylinders.

Dabei ist unter allgemeinem Zylinder der entsprechende mathematische Begriff zu verstehen, also die Fläche, die sich ergibt, wenn eine innerhalb einer Ebene liegende ebene Kurve entlang einer Strecke verschoben wird, die nicht in der Ebene enthalten ist.

Im Allgemeinen sind Stopfen bzw. Kanal senkrechte Zylinder, insbesondere senkrechte Kreiszylinder oder auch mit elliptischer oder polygonaler Grundform.

Entscheidend ist nur, dass das Verschlussmittel innerhalb des Kanals verschiebbar ist und ein Bereich des Verschlussmittels dichtend an der Innenwand des Kanals anliegt, damit die eingefüllte Flüssigkeit beim Verschieben nicht an dem Verschlussmittel vorbei austreten kann.

Entsprechend weist das Verschlussmittel bevorzugt an seinem Außenumfang ein Dichtmittel auf, insbesondere eine oder mehrere umlaufende Dichtlippen. Solche Dichtmittel sind bevorzugt aus einem elastomeren Material oder einem anderen Material, das bei Anpressung gegen die Innenwand eine geeignete Abdichtung erzielen kann.

Bevorzugt wird der Umfang der Verschiebung bzw. des Verschiebewegs des Verschlussmittels nach dem Befüllen nicht anhand von Erfahrungswerten ermittelt, sondern es ist ein Sensor vorgesehen oder auch mehrere Sensoren, mittels denen die Position einer Flüssigkeitsoberfläche einer in den Behälter eingefüllten Flüssigkeit relativ zur zweiten Öffnung bestimmbar ist. Alternativ oder zusätzlich kann der oder die Sensoren auch darauf ausgelegt sein, den Abstand zwischen der Flüssigkeitsoberfläche und der zweiten Öffnung bzw. das Unterschreiten eines definierten Abstands zwischen diesen zu bestimmen.

Dann kann das Verschieben abgebrochen werden, wenn entsprechend das unmittelbar vom Abstand zwischen Flüssigkeitsoberfläche und der zweiten Öffnung verbleibende mit Gas gefüllte Volumen ausreichend klein ist.

Bei einem Behälter, der einen Kopfbereich umfassend die zweite Öffnung aufweist, kann es ausreichend sein, wenn der Sensor so ausgelegt und/oder angeordnet ist, dass mittels des Sensors ein Vorhandensein der Flüssigkeit im Kopfbereich bestimmbar ist.

Allgemein kommen als Sensoren optische oder elektromagnetische Sensoren infrage. Denn sowohl optisch als auch elektromagnetisch gibt es zwischen den üblicherweise eingefüllten Flüssigkeiten und beispielsweise Raumluft erhebliche Differenzen, so dass die Messergebnisse entsprechender Sensoren zuverlässig ermitteln, ob nun an einer bestimmten Stelle des Volumens zwischen dem Verschlussmittel und der zweiten Öffnung die eingefüllte Flüssigkeit ist oder beispielsweise Raumluft. Durch geeignetes Platzieren eines oder mehrerer solcher Sensoren auf entsprechender Höhe bzw. in entsprechendem Abstand zur Position der zweiten Öffnung ist damit die Position der Flüssigkeitsoberfläche bzw. der Abstand dieser zur zweiten Öffnung zuverlässig bestimmbar.

Die kann insbesondere so erfolgen, dass ein Sensor nur auf einer einzigen Höhe bzw. in einem einzigen Abstand zu der zweiten Öffnung misst und entsprechend so lange das Verschlussmittel verschoben wird, bis dieser Sensor statt einem Raumgas die entsprechende Flüssigkeit registriert.

Bevorzugt umfasst der Sensor einen Sendeteil zum Aussenden eines optischen oder elektromagnetischen Signals und einen Empfangsteil zum Empfangen des Signals, die so angeordnet sind, dass der Behälter zwischen Sendeteil und Empfangsteil platziert ist. Dadurch lässt sich besonders präzise messen, ob die Flüssigkeit infolge der Verschiebung des Verschlussmittels bereits auf Höhe des Sensors ist oder noch nicht.

Entsprechend bevorzugt ist die Steuerung bei Verwendung eines Sensors ferner dazu ausgelegt, dass das Verschieben des Verschlussmittels entlang des Kanals bei einer bestimmten durch den Sensor bestimmten Position der Flüssigkeitsoberfläche durch entsprechende Ansteuerbefehle zu stoppen ist.

Es können aber auch Sensoren infrage kommen, die die Menge und/oder Ausdehnung von Gasen innerhalb des Behälters bestimmen, da entsprechend der Differenzbereich zwischen eingefüllter Flüssigkeit und zweiter Öffnung im Allgemeinen mit dem Gas ausgefüllt ist, das in dem Raum vorherrscht, in dem der Befüllvorgang abläuft.

Aus einer so gemessenen Gasmenge und der Behälterform ist unmittelbar die Position der Flüssigkeitsoberfläche oder deren Abstand zur zweiten Öffnung bestimmbar.

Das Schiebemittel ist bevorzugt ebenfalls mindestens teilweise in Form eines allgemeinen Zylinders ausgebildet, allerdings vorzugsweise mit einer kleineren Grundfläche als einer Grundfläche des allgemeinen Zylinders des Kanals.

Bevorzugt umfasst die Verschlussbetätigungsvorrichtung einen Linearmotor zum Bewegen des Schiebemittels.

Bevorzugt umfasst das Schiebemittel einen Kopfbereich, der korrespondierend zu einem Anordnungsbereich des Verschlussmittels ist, so dass das Verschlussmittel auf dem Schiebemittel positionierbar ist und durch das Schiebemittel in die erste Öffnung einbringbar ist.

Dies ist beispielsweise realisiert, indem das Schiebemittel kreiszylinderförmig ausgebildet ist und das Verschlussmittel eine untere kreiszylinderförmige Ausnehmung aufweist, womit es einfach auf dem Schiebemittel in seitlicher Richtung stabil positionierbar ist.

Bevorzugt umfasst das System ferner eine Wiegevorrichtung, mittels derer das Gewicht des Behälters vor, während und/oder nach dem Befüllen bestimmbar ist.

Bevorzugt umfasst das System ferner eine Halterungsvorrichtung, mittels derer Behälter in der Füllposition und/oder einer Position zum Verschieben des Verschlussmittels halterbar sind. Bevorzugt stimmt dabei die Position zum Verschieben des Verschlussmittels mit der Befüllposition überein.

Die Wiegevorrichtung ist dabei vorteilhaft, um eine Kontrolle der genauen Befüllmenge zu haben.

Bevorzugt wird das Verschiebemittel in der Behälterposition verschoben, in der der Behälter auch befüllt wird, da ein Bewegen des Behälters mit offener zweiter Öffnung eine weniger bevorzugte Variante ist.

Es kann aber auch vorgesehen sein, dass das Schiebemittel der Verschlussbetätigungsvorrichtung entfernt von anderen Mitteln der Verschlussbetätigungsvorrichtung angeordnet ist, die das Verschlussmittel beispielsweise in der Befüllposition des Behälters in der ersten Öffnung platzieren.

Nach anschließendem Befüllen des Behälters wird der Behälter entsprechend passend zum Schiebemittel oder umgekehrt positioniert und das Verschlussmittel wird mithilfe des Schiebemittels in der genannten Weise verschoben.

Bevorzugt umfasst die Verschlussbetätigungsvorrichtung weitere Mittel bzw. ist dazu ausgelegt, die zweite Öffnung des Behälters mit einem zweiten und bevorzugt nur gasdurchlässigen Verschlussmittel zu verschließen.

Dann ist es im Prinzip möglich, auf einen Sensor oder einen genauer bestimmten Verschiebeweg zu verzichten, da entsprechend verschoben wird, bis alles Gas aus dem Volumen zwischen Verschlussmittel und zweiter Öffnung verschwunden ist. Anschließend ist für einen gasdichten Verschluss an der zweiten Öffnung zu sorgen.

Bevorzugt umfasst die Verschlussbetätigungsvorrichtung einen Kraftsensor zur Bestimmung der durch das Schiebemittel ausgeübten Kraft. Ferner ist bevorzugt die Steuervorrichtung dazu ausgelegt, die Verschlussbetätigungsvorrichtung dazu anzusteuern, das Verschieben des Verschlussmittels einzustellen, wenn die durch das Schiebemittel ausgeübte Kraft einen bestimmten Grenzwert erreicht oder übersteigt.

Alternativ oder zusätzlich kann eine solche Kraft auch aus den Betriebsdaten des Antriebs des Schiebemittels bestimmbar sein.

Insbesondere in Kombination mit einem entsprechenden nur oder hauptsächlich nur gasdurchlässigen Verschluss der zweiten Öffnung kann mittels einer Kraftbestimmung der vom Schiebemittel ausgeübten Kraft zusätzlich oder alternativ zu den anderen hier genannten Methoden oder Vorrichtung eine geeignete Verringerung des Volumens zwischen Verschlussmittel und zweiter Öffnung bzw. ein geeigneter Verschiebeweg des Verschlussmittels gesteuert werden.

Die Erfindung betrifft ebenfalls ein Verfahren zum Befüllen eines Behälters mit mindestens zwei Öffnungen, wobei sich bei einer ersten der mindestens zwei Öffnungen ein Kanal mit einer Innenwand in der Form eines allgemeinen Zylinders anschließt, umfassend die Schritte:
a1) Positionieren des Behälters in einer Befüllposition, so dass die erste Öffnung des Behälters tiefer positioniert ist als wenigstens eine zweite der mindestens zwei Öffnungen des Behälters;
a2) Verschließen der ersten Öffnung des Behälters mit einem Verschlussmittel, wobei das Verschlussmittel wenigstens in einem Teilbereich einen Außenumfang aufweist, der so gestaltet ist, dass das Verschlussmittel beim Verschließen mit seinem Außenumfang dichtend an der Innenwand des Kanals zur Anlage kommt und so dass das Verschlussmittel entlang des Kanals bewegbar ist;
wobei a1) und a2) in beliebiger Reihenfolge ausgeführt werden;
c) Befüllen des Behälters mit einem definierten Volumen einer Flüssigkeit, das geringer ist als ein Volumen zwischen dem Verschlussmittel und der zweiten Öffnung des Behälters;
d) Bewegen des Verschlussmittels entlang des Kanals, so dass sich ein Volumen zwischen dem Verschlussmittel und der zweiten Öffnung des Behälters verkleinert.

Auf diese Weise ist die Präzision bei der initialen Positionierung des Verschlussmittels in der ersten Öffnung nicht mehr maßgeblich hinsichtlich der genau im Behälter enthaltenen Menge, weshalb eine Mehrbefüllung zum Toleranzausgleich hierfür nicht mehr erforderlich ist. Damit können mit derselben Menge Flüssigkeit bzw. Substanz mehr Behälter so befüllt werden, dass sie den entsprechend zu garantierenden Nominalwert mindestens enthalten und abgeben können.

Bevorzugt wird in Schritt d) die Bewegung des Verschlussmittels gestoppt, bevor oder wenn die Differenz definiertes Volumen minus Volumen zwischen dem Verschlussmittel und der zweiten Öffnung des Behälters einen definierten absoluten oder bezogen auf das Volumen zwischen dem Verschlussmittel und der zweiten Öffnung des Behälters relativen Wert erreicht oder unterschreitet. Der zu erreichende Wert der Differenz kann hierbei auch Null sein, also dass das Verschlussmittel solange verschoben wird, bis die Oberfläche der eingefüllten Flüssigkeit die zweite Öffnung erreicht.

Allerdings kann auch ein vorheriges Stoppen sinnvoll sein, je nachdem wie die zweite Öffnung im Weiteren verschlossen wird. Im Falle, dass die zweite Öffnung mit einem weiteren Verschlussmittel verschlossen werden soll, das in die zweite Öffnung hinein ragt, ist alternativ oder zusätzlich unter dem Volumen bzw. dem Abstand zwischen dem Verschlussmittel und der zweiten Öffnung das Volumen bzw. der Abstand zwischen dem Verschlussmittel und dem Punkt bzw. der Ebene zu verstehen, bis zu dem das weitere Verschlussmittel in die zweiten Öffnung hineinragen wird, insbesondere in dichtender Weise.

Durch entsprechend geeignetes Abbrechen des Verschiebens kann erreicht werden, dass dann nach Verschließen der zweiten Öffnung keine oder nur eine unerhebliche Menge Gas innerhalb des Behälters verbleibt.

Beim erfindungsgemäßen Verfahren kann der Behälter zwischen Schritt c) und d) sowohl an seiner Position verbleiben als auch bewegt werden. Dies richtet sich hauptsächlich nach den technischen Möglichkeiten der entsprechenden Anlage sowie den Behältereigenschaften, ob es nun effizienter und technisch unkomplizierter ist, den Behälter für das Verschieben an eine andere Stelle zu bringen, damit bereits ein weiterer Behälter befüllt werden kann, oder ob eben das Verschieben gut an der Befüllposition realisierbar ist.

Bevorzugt wird vor dem Schritt d) und insbesondere bevorzugt unmittelbar davor und damit entsprechend nach dem Schritt c) der Schritt c2) ausgeführt, nämlich Anordnen und/oder Aktivieren eines Sensors.

Dabei umfasst der Schritt d) ferner:
Bestimmen mithilfe des Sensors der in dem Volumen zwischen dem Verschlussmittel und der zweiten Öffnung des Behälters verbleibenden Gasmenge;
Bestimmen der Position einer Flüssigkeitsoberfläche der Flüssigkeit relativ zur zweiten Öffnung; und/oder
Bestimmen des Unterschreitens eines definierten Abstandes zwischen der Flüssigkeitsoberfläche und der zweiten Öffnung,
wobei bevorzugt Schritt d) bei Erreichen/Unterschreiten einer bestimmten Menge verbleibenden Gases, insbesondere 0 l, bei Erreichen/Passieren einer bestimmten Position der Flüssigkeitsoberfläche relativ zur zweiten Öffnung und/oder bei Erreichen/Unterschreiten des definierten Abstandes zwischen der Flüssigkeitsoberfläche und der zweiten Öffnung beendet wird.

Durch Verwenden eines Sensors zum Bestimmen, ob das Verschieben beendet werden soll, lässt sich präzise und automatisiert der Schritt d) im richtigen Moment beenden. Damit ist die genaue Positionierung des Verschlussmittels beim initialen Einsetzen in die erste Öffnung vollkommen unerheblich, weshalb die Ungenauigkeit bei dieser Positionierung auch nicht die Wirkung hat, dass zwecks Sicherstellung der Nominalabgabemenge eine größere Menge aufgrund der mangelnden Präzision bei dieser Positionierung abgefüllt werden muss.

Bevorzugt wird beim erfindungsgemäßen Verfahren in Schritt c) das Befüllen des Behälters gestoppt, bevor das Volumen zwischen dem Verschlussmittel und der zweiten Öffnung vollständig gefüllt ist. Dies bedeutet im Umkehrschluss, dass das Verschlussmittel in der ersten Öffnung initial auf jeden Fall so zu positionieren ist, dass das verbleibende Volumen zwischen Verschlussmittel und zweiter Öffnung auf jeden Fall größer ist als die einzufüllende Flüssigkeitsmenge bzw. Flüssigkeitsvolumen. Dann ist sichergestellt, dass der Optimierungseffekt durch späteres Verschieben des Verschlussmittels wirklich erreicht wird. Bei einem gelegentlichen vollständigen Füllen des Volumens zwischen Verschlussmittel und zweiter Öffnung würde hingegen zumindest für die entsprechenden Behälter eine Optimierung nicht erreicht werden.

Bevorzugt werden vor dem Schritt c) des erfindungsgemäßen Verfahrens eine Wiegevorrichtung und/oder ein Sensor angeordnet, wobei der Sensor unter Umständen derselbe sein kann, der wie vorher beschrieben den Abstand oder die Position der Flüssigkeitsoberfläche bestimmt, wobei mittels der Wiegevorrichtung bzw. dem Sensor vor/während und/oder nach Schritt c) die Menge der eingefüllten Flüssigkeit bestimmt wird. Dies erhöht die Produktsicherheit durch zusätzliche Kontrolle der eingefüllten Flüssigkeitsmenge bzw. Flüssigkeitsvolumens.

Bevorzugt erfolgt dies, indem vor dem Schritt c) ein Nettogewicht des Behälters ohne Flüssigkeit aber mit angeordnetem Verschlussmittel und während oder nach Schritt c) ein Bruttogewicht des Behälters mit Flüssigkeit bestimmt wird. Aus dem Bruttogewicht und dem Nettogewicht lässt sich das Gewicht der eingefüllten Flüssigkeit und mit deren Dichte auch die Flüssigkeitsmenge bzw. das Flüssigkeitsvolumen bestimmen.

In einer Variante wird bei dem erfindungsgemäßen Verfahren die zweite Öffnung vor dem Schritt d) und nach dem Schritt c) mit einem zweiten aber nur gasdurchlässigen Verschlussmittel verschlossen. Bei einem entsprechenden geeigneten Verschlussmittel erlaubt dies, den Schritt d) einfach zu beenden, wenn alles Gas zwischen Flüssigkeit und dem zweiten Verschlussmittel ausgetreten ist, was an entsprechend anderen Kraftverhältnissen oder anderem Verhalten des Antriebs des Schiebemittels ersichtlich ist.

Entsprechend kann auch in dieser Ausführungsform oder auch in anderen eine Bestimmung der vom Schiebemittel ausgeübten Kraft erfolgen, um anhand veränderter Kraftverhältnisse Rückschlüsse auf die Notwenigkeit der Beendigung des Verschiebens zu ziehen.

Bevorzugt werden die genannten Schritte in alphabetischer und dazu untergeordnet in numerischer Reihenfolge ausgeführt, z. B. a1), a2), c) und dann d).

Die vorliegende Erfindung wird nachfolgend anhand eines bevorzugten Ausführungsbeispiels unter Bezugnahme auf die beigefügten Zeichnungen näher erläutert. Dabei sind alle offenbarten Merkmale, die in Kombination mit dem Merkmal des Anspruchs 1 oder des Anspruchs 15 technisch realisiert werden können, als Teil der Erfindung anzusehen.

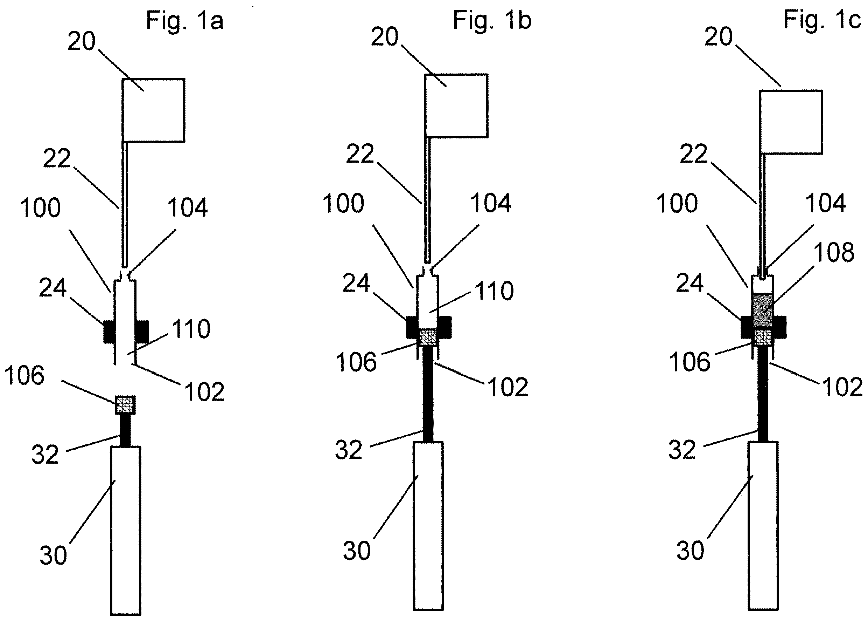

Figuren 1a bis 1f zeigen in einer schematischen Ansicht ein erfindungsgemäßes System sowie die Schritte eines erfindungsgemäßen Verfahrens zum Befüllen eines Behälters mit Nachschieben eines Verschlussmittels.

Wie in Figur 1a ersichtlich, umfasst ein erfindungsgemäßes System zum Befüllen von Behältern 100 mit mindestens zwei Öffnungen 102, 104 eine Befüllstation 20 mit einer Befüllvorrichtung 22 und Mitteln 24, beispielsweise eine Zange, zum Lagern eines Behälters 100 in einer Befüllposition.

Ferner umfasst das System eine Verschlussbetätigungsvorrichtung 30 mit Schiebemitteln 32, auf denen ein Verschlussmittel 106 angeordnet werden kann.

Dabei ist das System ausgelegt zum Befüllen von Behältern 100, bei denen sich bei einer ersten Öffnung 102 der mindestens zwei Öffnungen 102, 104 ein Kanal 110 mit einer Innenwand in der Form eines allgemeinen Zylinders anschließt.

Das Schiebemittel 32 kann dabei, wie in den Figuren 1b bis 1f gezeigt, verschoben werden, um das Verschlussmittel 106 geeignet zu positionieren. Das Verschlussmittel 106 ist dabei so ausgelegt, dass es die erste Öffnung 102 dichtend verschließt, so dass eine darüber gelagerte Flüssigkeit 108 nicht am Verschlussmittel 106 vorbei auslaufen kann. Ferner ist das Verschlussmittel 106 so dimensioniert, dass es auch ohne Stützung durch das Schiebemittel 32 in der ersten Öffnung 102 bzw. dem Kanal 110 dahinter gehalten ist.

Das Verschlussmittel 106 kann dabei wie gezeigt vollständig eine Form eines allgemeinen Zylinders entsprechend der Form des Kanals 110 aufweisen. Alternativ kann auch nur ein oberes Ende des Verschlussmittels 106 eine entsprechende Form aufweisen, während beispielsweise ein unteres Ende einen größeren Durchmesser als der Kanal 110 aufweist, so dass entsprechend nur das obere Ende des Verschlussmittels 106 innerhalb des Kanals 110 beweglich ist.

Die Befüllstation 20 oder wenigstens die Befüllvorrichtung 22 ist wie in Figur 1c gezeigt bevorzugt ebenfalls verschiebbar ausgebildet, so dass eine Absenkung zwecks Befüllen des Behälters durch die zweite Öffnung 104 möglich ist.

Das erfindungsgemäße System weist dabei eine Steuervorrichtung auf, die dafür ausgelegt ist, das erfindungsgemäße Verfahren auszuführen.

Dies umfasst als erstes den in Figur 1a und 1b gezeigten Schritt, dass der Behälter 100 mit der ersten Öffnung 102 unterhalb der zweiten Öffnung 104 liegend platziert wird, damit mithilfe des Schiebemittels 32 das Verschlussmittel 106, welches hier die Form eines Stopfens aufweist, in der ersten Öffnung 102 platziert werden kann.

Anschließend wird zwischen Figur 1b und 1c die Befüllstation 20 so verfahren, dass die Befüllvorrichtung 22 geeignet in den Behälter 100 durch die zweite Öffnung 104 hineinragt.

Anschließend gibt die Befüllvorrichtung 22 eine Flüssigkeit 108 ab, die sich im Behälter 100 in dem Raum zwischen dem Verschlussmittel 106 und der zweiten Öffnung 104 sammelt. Figur 1c zeigt gerade den Zustand während des Befüllens, bei dem dieses Volumen zwischen Verschlussmittel 106 und zweiter Öffnung 104 zu einem erheblichen Teil noch nicht gefüllt ist.

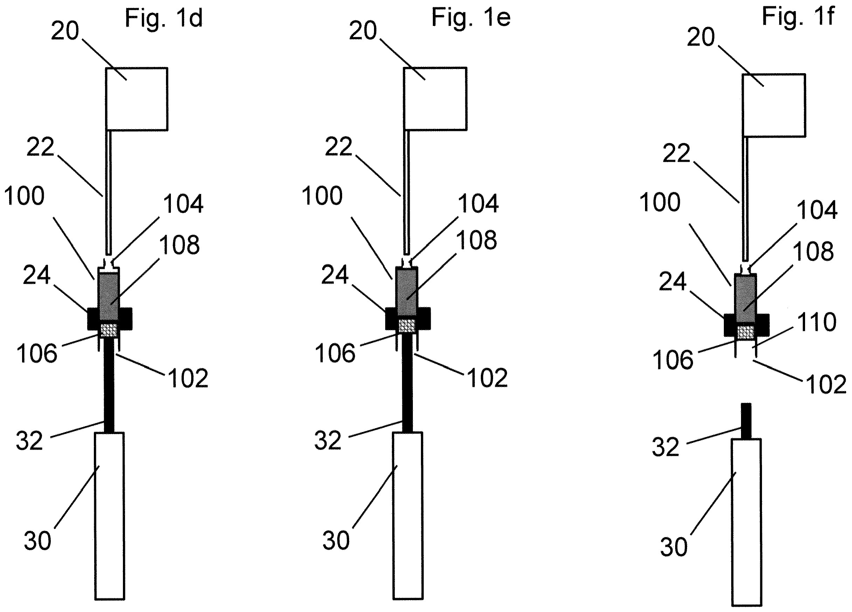

Wenn dieses Volumen bis zu einer gewünschten Höhe befüllt ist, werden Befüllstation 20 und Befüllvorrichtung 22 entsprechend wieder nach oben bewegt, so dass sich der in Figur 1d gezeigte Zustand einstellt.

Anschließend wird mithilfe des Schiebemittels 32 das Verschlussmittel bzw. der Stopfen 106 erneut geschoben bzw. nachgedrückt, um das zwischen der Öffnung 104 und der Oberfläche der Flüssigkeit 108 verbleibende Volumen zu verringern. Die Beendigung dieses Nachdrückens kann dabei wie oben geschildert auf verschiedene Weise erfolgen, beispielsweise mit einem nicht gezeigten Sensor, der entsprechend feststellt, wenn die Flüssigkeitsoberfläche der Flüssigkeit 108 die Stelle der Verengung unterhalb der Öffnung 104 erreicht.

Es stellt sich dann eine Situation wie in Figur 1e gezeigt ein.

Da das Verschlussmittel 106 rein durch die Innenwand des Kanals 110 ausreichend gehaltert ist, kann das Schiebemittel 32 nachfolgend einfach nach unten weg gezogen werden, womit sich der in Figur 1f gezeigte Zustand einstellt. Zur Positionierung des Verschlussmittels 106 an dem Schiebemittel 32 kann dies dabei insbesondere eine nicht gezeigte untere Ausnehmung aufweisen, in die die Oberseite des Schiebemittels 32 einfach eingeschoben wird, so dass das Schiebemittel 32 das Verschlussmittel 106 in seitlicher Richtung positioniert und dieses nach oben verschieben kann, aber gleichzeitig wie in Figur 1f gezeigt einfach nach unten weg gezogen werden kann.

In bekannter Weise und deshalb hier nicht weiter gezeigt, wird nachfolgend auf Figur 1f die zweite Öffnung 104 verschlossen. Da dabei das entsprechende Verschlussmittel ebenfalls ein gewisses Volumen aufweist, kann der Behälter 100 genau so verschlossen werden, dass die verbleibende Gasmenge Null oder geeignet gering ist. Gleichzeitig ist vollständig vermieden, dass eine zusätzliche Menge Flüssigkeit eingefüllt werden muss, um der Packmitteltoleranz hinsichtlich der nicht sonderlich präzisen initialen Positionierung des Verschlussmittels 106 Genüge zu tun.

## Patentansprüche

1. System zum Befüllen von Behältern (100) mit mindestens zwei Öffnungen (102, 104), wobei sich bei einer ersten (102) der mindestens zwei Öffnungen (102, 104) ein Kanal (110) mit einer Innenwand in der Form eines allgemeinen Zylinders anschließt, insbesondere Spritzen, Karpullen oder ähnliches, umfassend:
eine Befüllstation (20) mit einer Befüllvorrichtung (22) und Mitteln (24) zum Lagern eines Behälters (100) in einer Befüllposition, in der der Behälter (100) mittels der Befüllvorrichtung (22) befüllbar ist;
eine Verschlussbetätigungsvorrichtung (30), die ausgelegt ist, die erste Öffnung (102) des Behälters (100) mit einem Verschlussmittel (106) zu verschließen, wobei die erste Öffnung (102) in der Befüllposition tiefer positioniert ist als wenigstens eine zweite (104) der mindestens zwei Öffnungen (102, 104) des Behälters (100); und
eine Steuervorrichtung, die ausgelegt ist, Ansteuersignale für Schritte eines Befüllvorgangs abzugeben, wobei das Verschlussmittel (106) wenigstens in einem Teilbereich einen Außenumfang aufweist, der so gestaltet ist, dass das Verschlussmittel (106) beim Verschließen mit seinem Außenumfang dichtend an der Innenwand des Kanals (110) zur Anlage kommt und so dass das Verschlussmittel (106) entlang des Kanals (110) bewegbar ist
**dadurch gekennzeichnet, dass**
die Verschlussbetätigungsvorrichtung (30) Schiebemittel (32) umfasst, mittels derer eine auf ein bereits in der ersten Öffnung (102) angeordnetes Verschlussmittel (106) wirkende Kraft ausübbar ist, um das Verschlussmittel (106) entlang des Kanals (110) weiter zu bewegen;
die Steuervorrichtung ferner dazu ausgelegt ist, ein Befüllen des Behälters (100) nach einem definierten Volumen zu beenden, das geringer ist als ein Volumen zwischen dem Verschlussmittel (106) und der zweiten Öffnung (104) des Behälters (100); und dass
die Steuervorrichtung ferner dazu ausgelegt ist, die Verschlussbetätigungsvorrichtung (30) dazu anzusteuern, nach einem Befüllen des Behälters (100) mittels der Befüllvorrichtung (22) das Verschlussmittel (106) entlang des Kanals (110) mittels des Schiebemittels (32) zu verschieben, so dass sich ein Volumen zwischen dem Verschlussmittel (106) und der zweiten Öffnung (104) des Behälters (100) verkleinert.

2. System nach Anspruch 1, **dadurch gekennzeichnet, dass**
die Steuervorrichtung ferner dazu ausgelegt ist, die Ansteuerung der Verschlussbetätigungsvorrichtung (30), das Verschlussmittel (106) entlang des Kanals (110) zu verschieben, einzustellen bevor oder wenn die Differenz definiertes Volumen minus Volumen zwischen dem Verschlussmittel (106) und der zweiten Öffnung (104) des Behälters (100) einen definierten absoluten oder bezogen auf das Volumen zwischen dem Verschlussmittel (106) und der zweiten Öffnung (104) des Behälters (100) relativen Wert erreicht oder unterschreitet.

3. System nach einem der vorhergehenden Ansprüche, **dadurch gekennzeichnet, dass**
das Verschlussmittel (106) ein Stopfen in Form eines allgemeinen Zylinders ist.

4. System nach einem der vorhergehenden Ansprüche, **dadurch gekennzeichnet, dass**
das Verschlussmittel (106) an seinem Außenumfang ein Dichtmittel, insbesondere eine oder mehrere umlaufende Dichtlippen aufweist, bevorzugt aus einem elastomeren Material.

5. System nach einem der vorhergehenden Ansprüche, **dadurch gekennzeichnet, dass**
das System ferner einen Sensor umfasst, mittels dem die Position einer Flüssigkeitsoberfläche einer in den Behälter (100) eingefüllten Flüssigkeit (108) relativ zur zweiten Öffnung (104) bestimmbar ist und/oder mittels dem das Unterschreiten eines definierten Abstandes zwischen der Flüssigkeitsoberfläche und der zweiten Öffnung (104) bestimmbar ist.

6. System nach Anspruch 5, **dadurch gekennzeichnet, dass**
der Behälter (100) einen die zweite Öffnung (104) umfassenden Kopfbereich aufweist, wobei der Sensor so ausgelegt und/oder angeordnet ist, dass mittels des Sensors ein Vorhandensein der Flüssigkeit im Kopfbereich bestimmbar ist.

7. System nach Anspruch 5 oder 6, **dadurch gekennzeichnet, dass**
der Sensor ein optischer oder elektromagnetischer Sensor ist, wobei der Sensor bevorzugt einen Sendeteil zum Aussenden eines optischen oder elektromagnetischen Signals und einen Empfangsteil zum Empfangen des Signals umfasst, die so angeordnet sind, dass der Behälter (100) zwischen Sendeteil und Empfangsteil platzierbar ist.

8. System nach einem der Ansprüche 5, 6 oder 7, **dadurch gekennzeichnet, dass**
die Steuerung ferner dazu ausgelegt ist, das Verschieben des Verschlussmittels (106) entlang des Kanals (110) bei einer festgelegten, durch den Sensor bestimmten Position der Flüssigkeitsoberfläche zu stoppen.

9. System nach einem der Ansprüche 5 bis 8, **dadurch gekennzeichnet, dass**
mittels dem Sensor die Position der Flüssigkeitsoberfläche und/oder deren Abstand zur zweiten Öffnung (104) bestimmbar ist, indem der Sensor Menge und/oder Ausdehnung von Gasen innerhalb des Behälters (100) bestimmt.

10. System nach einem der vorhergehenden Ansprüche, **dadurch gekennzeichnet, dass**
das Schiebemittel (32) wenigstens teilweise in Form eines allgemeinen Zylinders ausgebildet ist mit einer kleineren Grundfläche als einer Grundfläche des allgemeinen Zylinders des Kanals (110); und/oder
die Verschlussbetätigungsvorrichtung (30) einen Linearmotor zum Bewegen des Schiebemittels (32) umfasst.

11. System nach einem der vorhergehenden Ansprüche, **dadurch gekennzeichnet, dass**
das Schiebemittel (32) einen Kopfbereich umfasst, der korrespondierend zu einem Anordnungsbereich des Verschlussmittels (106) ist, so dass das Verschlussmittel (106) auf dem Schiebemittel (32) positionierbar ist und durch das Schiebemittel (32) in die erste Öffnung (102) einbringbar ist.

12. System nach einem der vorhergehenden Ansprüche, **dadurch gekennzeichnet, dass**
das System ferner eine Wiegevorrichtung umfasst, mittels derer das Gewicht des Behälters (100) vor, während und/oder nach dem Befüllen bestimmbar ist, und/oder
das System ferner eine Halterungsvorrichtung umfasst, mittels derer der Behälter (100) in der Befüllposition und/oder einer Position zum Verschieben des Verschlussmittels (106) halterbar ist, wobei bevorzugt die Position zum Verschieben des Verschlussmittels (106) mit der Befüllposition übereinstimmt.

13. System nach einem der vorhergehenden Ansprüche, **dadurch gekennzeichnet, dass**
die Verschlussbetätigungsvorrichtung (30) ferner dazu ausgelegt ist, die zweite Öffnung (104) des Behälters (100) mit einem zweiten nur gasdurchlässigen Verschlussmittel zu verschließen.

14. System nach einem der vorhergehenden Ansprüche, **dadurch gekennzeichnet, dass**
die Verschlussbetätigungsvorrichtung (30) einen Kraftsensor umfasst zur Bestimmung der durch das Schiebemittel (32) ausgeübten Kraft, wobei bevorzugt die Steuervorrichtung ferner dazu ausgelegt ist, die Verschlussbetätigungsvorrichtung (30) dazu anzusteuern, das Verschieben des Verschlussmittels (106) einzustellen, wenn die durch das Schiebemittel (32) ausgeübte Kraft einen bestimmten Grenzwert erreicht oder übersteigt.

15. Verfahren zum Befüllen eines Behälters (100) mit mindestens zwei Öffnungen (102, 104), wobei sich bei einer ersten (102) der mindestens zwei Öffnungen (102, 104) ein Kanal (110) mit einer Innenwand in der Form eines allgemeinen Zylinders anschließt, umfassend die Schritte:
a1) Positionieren des Behälters (100) in einer Befüllposition, so dass die erste Öffnung (102) des Behälters (100) tiefer positioniert ist als wenigstens eine zweite (104) der mindestens zwei Öffnungen (102, 104) des Behälters (100);
a2) Verschließen der ersten Öffnung (102) des Behälters (100) mit einem Verschlussmittel (106), wobei das Verschlussmittel (106) wenigstens in einem Teilbereich einen Außenumfang aufweist, der so gestaltet ist, dass das Verschlussmittel (106) beim Verschließen mit seinem Außenumfang dichtend an der Innenwand des Kanals (110) zur Anlage kommt und so dass das Verschlussmittel (106) entlang des Kanals (110) bewegbar ist;
wobei a1) und a2) in beliebiger Reihenfolge ausgeführt werden;
c) Befüllen des Behälters (100) mit einem definierten Volumen einer Flüssigkeit (108), das geringer ist als ein Volumen zwischen dem Verschlussmittel (106) und der zweiten Öffnung (104) des Behälters (100);
d) Bewegen des Verschlussmittels (106) entlang des Kanals (110), so dass sich ein Volumen zwischen dem Verschlussmittel (106) und der zweiten Öffnung (104) des Behälters (100) verkleinert.

16. Verfahren zum Befüllen eines Behälters (100) nach Anspruch 15, wobei im Schritt d) die Bewegung des Verschlussmittels (106) gestoppt wird, bevor oder wenn die Differenz definiertes Volumen minus Volumen zwischen dem Verschlussmittel (106) und der zweiten Öffnung (104) des Behälters (100) einen festgelegten absoluten oder bezogen auf das Volumen zwischen dem Verschlussmittel (106) und der zweiten Öffnung (104) des Behälters (100) relativen Wert erreicht oder unterschreitet.

17. Verfahren zum Befüllen eines Behälters (100) nach einem der vorhergehenden auf ein Verfahren gerichteten Ansprüche, wobei der Behälter (100) zwischen Schritt c) und d) bewegt oder nicht bewegt wird.

18. Verfahren zum Befüllen eines Behälters (100) nach einem der vorhergehenden auf ein Verfahren gerichteten Ansprüche, wobei die Position des Behälters (100) bei Schritt c) und d) identisch oder verschieden ist.

19. Verfahren zum Befüllen eines Behälters (100) nach einem der vorhergehenden auf ein Verfahren gerichteten Ansprüche, wobei vor dem Schritt d) und bevorzugt unmittelbar davor und nach dem Schritt c) der Schritt ausgeführt wird:
c2) Anordnen und/oder Aktivieren eines Sensors; wobei der Schritt d) ferner umfasst:
Bestimmen mithilfe des Sensors der in dem Volumen zwischen dem Verschlussmittel (106) und der zweiten Öffnung (102, 104) des Behälters (100) verbleibenden Gasmenge;
Bestimmen der Position einer Flüssigkeitsoberfläche der Flüssigkeit (108) relativ zur zweiten Öffnung (104); und/oder
Bestimmen des Unterschreitens eines definierten Abstandes zwischen der Flüssigkeitsoberfläche und der zweiten Öffnung (104),
wobei bevorzugt Schritt d) bei Erreichen/Unterschreiten einer bestimmten Menge verbleibenden Gases, insbesondere 0 l, bei Erreichen/Passieren einer bestimmten Position der Flüssigkeitsoberfläche relativ zur zweiten Öffnung (104) und/oder bei Erreichen/Unterschreiten des definierten Abstandes zwischen der Flüssigkeitsoberfläche und der zweiten Öffnung (104) beendet wird.

20. Verfahren zum Befüllen eines Behälters (100) nach einem der vorhergehenden auf ein Verfahren gerichteten Ansprüche, wobei in Schritt c) das Befüllen des Behälters (100) gestoppt wird, bevor das Volumen zwischen dem Verschlussmittel (106) und der zweiten Öffnung (104) vollständig gefüllt ist.

21. Verfahren zum Befüllen eines Behälters (100) nach einem der vorhergehenden auf ein Verfahren gerichteten Ansprüche, wobei eine Wiegevorrichtung und/oder ein Sensor vor dem Schritt c) angeordnet werden, und/oder nach Anspruch 18, wobei mittels der Wiegevorrichtung bzw. dem Sensor vor/ während und/oder nach Schritt c) die Menge der eingefüllten Flüssigkeit bestimmt wird, bevorzugt indem vor dem Schritt c) ein Nettogewicht des Behälters (100) ohne Flüssigkeit aber mit angeordnetem Verschlussmittel (106) und während Schritt c) ein Bruttogewicht des Behälters (100) mit Flüssigkeit bestimmt wird.

22. Verfahren zum Befüllen eines Behälters (100) nach einem der vorhergehenden auf ein Verfahren gerichteten Ansprüche, wobei die zweite Öffnung (104) vordem Schritt d) und nach dem Schritt c) mit einem zweiten nur gasdurchlässigen Verschlussmittel verschlossen wird, wobei bevorzugt das Bewegen in Schritt d) beendet wird, wenn kein Gas mehr zwischen Flüssigkeit (108) und dem zweiten Verschlussmittel ist.
